# EUROPEAN PATENT APPLICATION

(11) **EP 4 755 900 A1**
(43) Date of publication of application: **10.06.2026**
(21) Application number: 24849299.3
(22) Date of filing: 02.08.2024
(51) Int. Cl.: C07K 14/20, C12N 15/31, G01N 33/571

(54) **IMMUNOASSAY METHOD USING TREPONEMA PALLIDUM ANTIGEN 17 KDA (TPN17) AS ANTIGEN**

(30) Priority: 02.08.2023 JP 2023126467
(71) Applicant: Denka Company Limited, Tokyo 103-8338 (JP)
(72) Inventor: YANAGITA Tomoyo, Tokyo 103-8338 (JP); JARCZOWSKI Franziska, 06120 Halle/ Saale (DE); DIESSNER André, 06120 Halle/ Saale (DE)
(74) Representative: Hoffmann Eitle
(86) International application number: PCT/JP2024/027732
(87) International publication number: WO 2025/028654

(57) **Abstract**

This invention provides a method of immunoassay comprising detecting an antibody specific to *Treponema pallidum* in the serum with the use of the *Treponema pallidum* 17 kDa antigen (TpN17) as the antigen. This invention also provides a protein comprising a sequence deleted a part of the amino acid sequence of TpN17, and a method of immunoassay involving the use of such protein as the antigen.

## Description

### Technical Field

The present invention relates to an immunoanalytical method using the *Treponema pallidum* 17 kDa antigen (TpN17) as the antigen, a reagent used therefor, and a method using the reagent.

### Background Art

*Treponema pallidum* belongs to the bacterial family of spirochetes and is the causative pathogen of syphilis. Syphilis is primarily transmitted through sexual contact and may also be transmitted from the mother to the baby during pregnancy. The disease is characterized by distinct clinical stages and a long period of latent asymptomatic infection. Many people do not notice the symptoms and therefore remain unaware of their syphilis infection for years.

Effective treatments have been available since penicillin was introduced in the mid-20th century; however, syphilis remains a critical health problem around the world. In order to aid antibiotic therapy and prevent prevalence of syphilis, it is essential to identify patients infected with *Treponema pallidum.* In order to detect antibodies to *Treponema pallidum* in the serum, immunoassay or other diagnostic tools have been used.

Concerning serum antibodies after infection, the antibody titers specific to cardiolipin (tests using non-treponemal antigens: VDRL, the RPR card method, and the RPR automated method) are first elevated, and the antibody titers specific to *Treponema pallidum* (tests using *Treponema pallidum* antigens: the FTA-ABS method, the TPHA method, the TPPA method, and the TP antibody automated method) are then elevated. The anti-cardiolipin antibody titer is lowered in response to the treatment, and it is thus used for evaluation of treatment effects. However, cardiolipin is not a specific antigen, and, accordingly, biological false-positive reactions may occur. While the specificity of the *anti-Treponema pallidum* antibody assay is high, the antibody titer is gradually lowered but is continuously evaluated positive after the treatment. Accordingly, it is difficult to distinguish the latest syphilis infection from past syphilis infections.

### Citation List

### Patent Literature

Patent Literature 1: JP Patent Publication (Kokai) No. H09-288110 A

### Non Patent Literature

Non Patent Literature 1: V. Sambri et al., Clin. Diagn. Lab. Immunol., May 2001; 8 (3): 534-9

### Summary of Invention

### Technical Problem

An object of the present invention is to provide an immunoassay method for detecting a specific antibody to *Treponema pallidum* in the serum with the use of the *Treponema pallidum* 17 kDa antigen (TpN17) as the antigen. TpN17 is capable of detecting the *anti-Treponema pallidum* antibodies, which are IgG and IgM antibodies. In order to easily distinguish the latest syphilis infection from past syphilis infections, it is considered necessary to further enhance the assay sensitivity to IgM that is elevated at an early stage of syphilis infection. If the assay sensitivity to IgM that is elevated at an early stage of syphilis infection can further be enhanced, syphilis infection may be detected at an early stage. Accordingly, an object of the present invention is to provide an immunoassay method for detecting a specific IgM antibody to *Treponema pallidum* in the serum with the use of the *Treponema pallidum* 17 kDa antigen (TpN17) as the antigen.

### Solution to Problem

The present inventors have conducted concentrated studies. As a result, they discovered that deprivation of a part of the amino acid sequence of the TpN17 antigen would enhance the reactivity with the *anti-Treponema pallidum* IgM antibody. This has led to the completion of the present invention.

Specifically, the present invention is as described below.
[1] A protein comprising an amino acid sequence of TpN17, comprising deletion of a part of the amino acid sequence.
[2] The protein according to [1], wherein the deletion of a part of the amino acid sequence of TpN17 is deletion of 1 to 20 amino acids at the N terminus of the amino acid sequence shown in SEQ ID NO: 1.
[3] The protein according to [2] comprising the amino acid sequence shown in SEQ ID NO: 3, wherein the deletion of a part of the amino acid sequence of TpN17 is deletion of 11 amino acids at the N terminus.
[4] A TpN17 antigen comprising the protein according to any of [1] to [3].
[5] A method for measuring an *anti-Treponema pallidum* antibody by an immunoassay method with the use of the protein according to any of [1] to [3] or the TpN17 antigen according to [4] as the antigen.
[6] The method according to [5], comprising measuring an *anti-Treponema pallidum* IgM antibody with high sensitivity.
[7] The method according to [5] or [6], which is an agglutination method.
[8] A kit for measuring an anti-*Treponema pallidum* antibody by an immunoassay method, the kit comprising the protein according to any of [1] to [3] or the TpN17 antigen according to [4] as the assay antigen for measurement.
[9] The kit according to [8], wherein the immunoassay method is an agglutination method.
The description incorporates the contents disclosed by JP Patent Application No. 2023-126467, based on which the priority of the present application claims.

### Advantageous Effects of Invention

The *anti-Treponema pallidum* antibody may be measured with the use of the TpN17 antigen of the present invention, so as to measure the *anti-Treponema pallidum* IgM antibody with high sensitivity. This enables detection of syphilis infection at an early stage of infection with high sensitivity.

### Description of Embodiments

Hereafter, the present invention is described in detail. The unit "%" used herein is by mass (w/v%), unless otherwise specified.

The present invention relates to a protein in which a part of the amino acid sequence of the *Treponema pallidum* 17 kDa surface antigen (TpN17) is deleted.

*Treponema pallidum* is known to comprise surface antigens with a wide variety of molecular weights on the cell surface thereof, and antigens with molecular weights of 47 kDa, 42 kDa, 17 kDa, and 15 kDa are known as major antigens. Such antigens are clearly separated from each other by electrophoresis. In addition to the gene encoding the 47 kDa surface antigen, the genes encoding 17 kDa and 15 kDa surface antigens have already been cloned, and antigens have been produced by genetic engineering. In addition, the amino acid sequences thereof have been determined.

The protein of the present invention has achieved enhanced reactivity with the anti-*Treponema pallidum* IgM antibody as a result of deletion of a part of the amino acid sequence of TpN17. As a result of deletion of a part of the amino acid sequence of TpN17, measurement values are increased. This enables assays of the *anti-Treponema pallidum* antibody with high sensitivity. With the use of a protein in which a part of the amino acid sequence of the *Treponema pallidum* 17 kDa surface antigen (TpN17) is deleted, specifically, the anti-*Treponema pallidum* IgM antibody can be measured with high sensitivity.

SEQ ID NO: 1 shows an amino acid sequence deleted the amino acid sequence of the signal peptide and the lipid-modified cysteine residue of the full-length amino acid sequence of TpN17, SEQ ID NO: 2 shows the full-length amino acid sequence of TpN17 (Swiss-Prot P29722), and SEQ ID NO: 3 shows the amino acid sequence of ΔTpN17 deprived of 11 amino acids from the N terminus of the amino acid sequence of TpN17 shown in SEQ ID NO: 1.

The amino acid sequences of SEQ ID NOs: 1 to 3 are shown below.
SEQ ID NO: 1 
VSCTTVCPHAGKAKAEKVECALKGGIFRGTLPAADCPGIDTTVTFNADGTAQKVELA
LEKKSAPSPLTYRGTWMVREDGIVELSLVSSEQSKAPHEKELYELIDSNSVRYMGAP
GAGKPSKEMAPFYVLKKTKK
SEQ ID NO: 2 
MKGSVRALCAFLGVGALGSALCVSCTTVCPHAGKAKAEKVECALKGGIFRGTLPAA
DCPGIDTTVTFNADGTAQKVELALEKKSAPSPLTYRGTWMVREDGIVELSLVSSEQS
KAPHEKELYELIDSNSVRYMGAPGAGKPSKEMAPFYVLKKTKK
SEQ ID NO: 3

KAKAEKVECALKGGIFRGTLPAADCPGIDTTVTFNADGTAQKVELALEKKSAPSPLT
YRGTWMVREDGIVELSLVSSEQSKAPHEKELYELIDSNSVRYMGAPGAGKPSKEMAP
FYVLKKTKK

The TpN17 protein used as an antigen for measurement in the present invention does not comprise the amino acid sequence of the signal peptide (amino acids 1 to 19) and the lipid-modified cysteine residue (20th amino acid) in the full-length amino acid sequence of TpN17 shown in SEQ ID NO: 2.

Deletion of a part of the amino acid sequence of TpN17 is deletion of 1 to 20 amino acids, preferably 5 to 15 amino acids, more preferably 7 to 14 amino acids, more preferably 8 to 13 amino acids, more preferably 9 to 12 amino acids, and particularly preferably 11 amino acids from the N terminus of the amino acid sequence shown in SEQ ID NO: 1. Specifically, the deletion of a part of the amino acid sequence is deletion of amino acids 1 to 42, 1 to 41, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, or 1 to 23 from the complete amino acid sequence of TpN17 shown in SEQ ID NO: 2. In particular, deletion of amino acids 1 to 33 is preferable.

The protein of the present invention can be obtained by incorporating DNA encoding a protein comprising a sequence deleted a part of the amino acid sequence of TpN17 into a plasmid vector, introducing the plasmid vector into a host cell, and expressing the protein.

A host cell is not particularly limited, provided that it can produce the protein of the present invention. Examples of host cells include insect cells (e.g., Sf9 and Hi5 cells), *E. coli* cells, yeast cells (e.g., *Saccharomyces cerevisiae, Saccharomyces pombe, Pichia pastoris),* mammalian cells (e.g., CHO and HEK cells), and any other cells.

The protein of the present invention can be prepared with the use of a plant expression system or a genetically engineered plant. Protein production using the transient expression system in plants can be performed in accordance with a conventional technique. Examples of such techniques include, but are not particularly limited to, the agroinfiltration method, the plant virus vector method, the magnICON^{®} system, and the particle gun method.

When the agroinfiltration method is employed, *Agrobacterium* may be transformed with the vector, and a plant may then be infected with the *Agrobacterium* to obtain a plant that expresses the protein.

In the present invention, a protein in which a part of the amino acid sequence of the *Treponema pallidum* 17 kDa surface antigen (TpN17) is deleted is referred to as the TpN17 antigen. Specifically, the present invention encompasses the TpN17 antigen consisting of a protein in which a part of the amino acid sequence of the *Treponema pallidum* 17 kDa surface antigen (TpN17) is deleted.

In addition, the present invention encompasses a method for measurement and immunoanalysis of anti-TpN17 antibody with the use of a protein in which a part of the amino acid sequence of the *Treponema pallidum* 17 kDa surface antigen (TpN17) is deleted as the antigen. Anti-TpN17 antibody measurement enables detection of the presence or absence of *Treponema pallidum* in a test sample obtained from a subject. By detecting the presence or absence of *Treponema pallidum* in a test sample, whether or not the subject is infected with *Treponema pallidum;* i.e., whether or not the subject has syphilis, can be determined, or auxiliary data used for diagnosis thereof can be obtained. With the use of a protein in which a part of the amino acid sequence of the *Treponema pallidum* 17 kDa surface antigen (TpN17) is deleted as the antigen, the *anti-Treponema pallidum* TpN17 IgM antibody that appears at an early stage of infection can be detected with high sensitivity, and syphilis can be detected at an early stage of infection.

The present invention can be applied to any immunological measurement methods that utilize the antigen-antibody reactions. Specifically, the present invention can be applied to, for example, immunodiffusion, immunoturbidimetry, immunonephelometry, hemagglutination, the latex method, enzyme immunoassay (EIA), radioimmunoassay (RIA), or immunochromatography. In particular, the present invention exerts remarkable effects in the agglutination methods, such as immunoturbidimetry, immunonephelometry, hemagglutination, and the latex agglutination method.

The agglutination method is an immunoassay method involving the use of insoluble carriers, such as latex particles, bentonite, collodion, kaolin, fixed sheep red blood cells, and the like. Insoluble carriers are preferably latex particles, and examples of latex particles that can be used include polystyrene latex particles, styrene-butadiene copolymer latex particles, and polyvinyl toluene latex particles. In immunoassays using the agglutination method, insoluble carriers may be sensitized with antigens; that is, the antigens may be bound to the surface of the carriers.

The immunoassay method of the present invention is preferably performed with the use of an automated analyzer. An automated analyzer is used for biochemical testing that can be applied to an immunoassay method, which comprises a mechanism of dispensing a reagent into a reaction vessel through a tube by a continuous dispensing system. More specifically, such automated analyzer comprises a means for providing a reagent inside or outside the apparatus and it continuously dispenses a reagent through a tube into a reaction vessel provided in the apparatus. The reagent is dispensed through the tube with the aid of a pump, such as a peristaltic pump, in combination with a switch valve. Examples of such apparatuses includes various commercially available apparatuses, such as Hitachi Automated Analyzer 7180, Hitachi Automated Analyzer 7250, Hitachi Automated Analyzer 7450, and Hitachi Automated Analyzer 7350 (Hitachi High-Tech Corporation).

A method of measurement performed with the use of an automated analyzer comprises optically capturing agglutinates resulting from bond formation between the antigen-sensitized carriers and the target antibodies in a reaction vessel and measuring the antibodies. Specifically, agglutinates are irradiated with lights in visible to near-infrared ranges, and a change in the absorbance or a change in the scattered light intensity is detected to measure the extent of agglutination of carrier particles. Examples of methods of measurement include the one-point method, the two-point method, the three-point method, and the rate method, and the method of the present invention can be applied to any of such methods of measurement. A test sample is dispensed into a reaction vessel in advance, the first reagent and the second reagent are dispensed thereinto, the reaction is allowed to proceed, and the extent of agglutination is measured. Thus, the target antigen or the target antibody in the test sample can be quantified using the extent of agglutination as the indicator. The order to add the first reagent and the second reagent is not limited. It is preferable that the first reagent be dispensed before the second reagent or the first reagent and the second reagent be dispensed simultaneously. In such a case, a standard curve relating the concentration of the target analyte to the extent of agglutination may be created in advance using a plurality of samples with known concentration of the target analyte, and the concentration of the target analyte in the test samples can be calculated based on the standard curve.

The first reagent may contain a buffer. The first reagent may contain another stabilizer reagent, such as a surfactant, a protective reagent, and the like. The second reagent may contain the particle sensitized with the antigen that binds specifically to the analyte antibody, and the second reagent may contain another stabilizer reagent, such as a surfactant, a protective reagent, and the like.

A method for sensitizing a carrier with an antigen may be performed in accordance with a conventional technique without particular limitation. For example, an antigen may be physically adsorbed to or chemically bound to a carrier. More specifically, for example, an antigen may be mixed with a carrier, and the mixture may be heated with shaking at 30°C to 37°C for 1 to 2 hours to sensitize the carrier with the antigen. The amount of the antigen to sensitize the carrier can be appropriately determined in accordance with the particle diameter of the carrier used. After the carrier is sensitized with the antigen, an unsensitized region on the carrier surface is preferably blocked with, for example, bovine serum albumin, human serum albumin, rabbit serum albumin, or egg albumin. The antigen-sensitized carrier is preferably retained in the form of a medium dispersion before the reaction with the test sample. Examples of media that can be used herein include phosphate buffer and glycine buffer. A medium may be supplemented with bovine serum albumin, gelatin, gum Arabic, or the like, according to need. The antigen-sensitized carrier thus prepared is allowed to react with the test sample, the reactivity between the sensitizing antigen and the antibody in the test sample is determined based on the presence or absence or the extent of agglutination, and the antibody in the test sample can be detected.

In the agglutination method using sensitized particles, detection is performed by adding several tens to several hundreds of µl of physiological saline or an adequate buffer to several to several tens of µl of the test sample, mixing the mixture in an adequate reaction vessel, performing incubation for several minutes, and adding several tens to several hundreds of µl of antigen-bound sensitized particles, followed by incubation for several minutes. Subsequently, the absorbance may be measured at an adequate wavelength (e.g., 570 nm) to measure the turbidity caused by agglutination of sensitized particles resulting from the antigen-antibody reaction. Alternatively, several tens to several hundreds of µl of a solution of sensitized particles suspended in a buffer may be added to several µl of a reaction sample. Examples of buffers include adequate buffers with pH 5.0 to 10, such as phosphate buffer, borate buffer, and Tris buffer.

The test samples subjected to the immunoassay method of the present invention are not particularly limited, body fluid samples, such as blood, serum, plasma, urine, stool, saliva, interstitial fluid, spinal fluid, and swab specimen samples, or a diluted product thereof are preferable, and blood, serum, and plasma samples or a diluted product thereof are more preferable.

In the agglutination method, the first reagent may be mixed with the second reagent containing latex particles.

The present invention further encompasses an immunoassay kit comprising the assay reagent. The immunoassay kit comprises a protein in which a part of the amino acid sequence of TpN17 is deleted as an antigen for measurement. When the buffer composition does not contain antigen-sensitized particles, the immunoassay kit may further comprise antigen-sensitized particles separately, and, furthermore, the kit may comprise a positive control, a negative control, instructions, and the like.

### Examples

The present invention is described in greater detail with reference to the following examples, although the present invention is not limited to these examples.

### (1) Preparation of TpN17

TpN17 was prepared using the transient expression system in plants. TpN17 was cloned into the tobacco mosaic virus (TMV) vector (Icon Genetics). The vector was transformed into an *Agrobacterium* cell, the transformed cell was cultured, the mixed culture was infiltrated into *Nicotiana benthamiana* leaves, the leaves were harvested approximately 1 week later, and the harvested leaves (infected leaves) were then frozen.

The frozen infected leaves (200 g) were separated by weighing and ground using a cutter mixer. An extraction solution (200 ml, 100 mM Tris, 250 mM NaCl, 5 mM EDTA, 40 mM sodium ascorbate) was added to the ground leaves, and the infected leaves were repeatedly ground. The supernatant was collected by centrifugation. The collected solution was filtered through a 0.22-µm filter. The filtrate was subjected to affinity column chromatography to remove impurities.

TpN17 (SEQ ID NO: 1) with deletion of the amino acid sequence of the signal peptide and the lipid-modified cysteine residue and ΔTpN17 (SEQ ID NO: 3) with deletion of a part of the amino acid sequence from the amino acid sequence of full-length TpN17 were expressed in the manner described above.

TpN15 and TpN47 were prepared in the same manner. The amino acid sequences of TpN15 and TpN47 and the DNA sequences encoding the same are known.

### (2) Preparation of first reagent

A surfactant and bovine serum albumin were added to a buffer (Tris, pH 8.0) to prepare a first reagent.

### (3) Preparation of second reagent

With the use of TpN17, ΔTpN17, TpN15, and TpN47 prepared in (1), assay reagents for immunoagglutination were prepared in the manner described below.

Sensitized particles carrying TpN17, ΔTpN17, TpN15, and TpN47 prepared in (1) in an amount of 3.9 mg relative to 1 ml of a polystyrene latex suspension were suspended in a buffer (Bis-Tris, pH 6.0) at 0.12% to prepare a second reagent.

### (4) Measurement using automated analyzer

Measurement was performed automatically by an end-point assay method using Hitachi Automated Analyzer 7180 (Hitachi High-Tech Corporation).

The first reagent and the second reagent containing TpN17- or ΔTpN17-sensitized particles were used to measure serum samples. The first reagent (100 µl) was added to 8.5 µl of the specimen solution, and the mixture was mixed at 37°C with stirring. The mixture was allowed to stand for 5 minutes, 50 µl of the second reagent was added thereto, and the mixture was further mixed at 37°C with stirring. The result of the agglutination reaction performed for approximately 5 minutes was measured as the change in the absorbance, and the anti-*Treponema pallidum* antibody concentration of each specimen was determined based on the calibration curve prepared using the standard solution (HEPES buffer, bovine serum albumin, sodium chloride, the *anti-Treponema pallidum* antibody, pH 7.4).

### (5) Comparison of reactivity of TpN17

The measurement values of the reagents for specimens were compared, the reagents using TpN17 with deletion of the amino acid sequence of the signal peptide and the lipid-modified cysteine residue (SEQ ID NO: 1) and using ΔTpN17 with deletion of a part of the amino acid sequence (SEQ ID NO: 3) from the amino acid sequence of complete TpN17 prepared in (1).

Serum specimens (2 specimens) obtained from syphilis patients were used. The specimens were measured in the manner described in (4) with the use of the first reagent prepared in (2) and the second reagent using TpN17 or ΔTpN17 prepared in (3).

The serum samples obtained from syphilis patients were measured to detect the anti-*Treponema pallidum* antibody in the human serum samples with the use of *recomLine* Treponema IgG (tradename, commercially available from MIKROGEN) and *recomLine* Treponema IgM (tradename, commercially available from MIKROGEN). Evaluation as to the presence or absence of antibodies to each of the *Treponema pallidum* antigens (TpN17, TpN47, and TpN15) and evaluation as to the intensities of the detected bands were made in accordance with the standards shown in Table 1 in comparison with cutoff bands on each of test papers.

**[Table 1]**

| Evaluation of band intensity in association with cutoff band | |
|---|---|
| Band staining intensity | Score |
| No reactivity | 0 |
| Very low intensity (lower than the cutoff band) | 0.5 |
| Low intensity (equal to the cutoff band) | 1 |
| High intensity (higher than the cutoff band) | 2 |
| Very high intensity | 3 |

The results are shown in Table 2.

**[Table 2]**

| | Measurement value (U/ml) | | IgM | | | IgG | | |
|---|---|---|---|---|---|---|---|---|
| | Complete TpN17 | ΔTpN17 | TpN47 | TpN17 | TpN15 | TpN47 | TpN17 | TpN15 |
| Specimen 1 | 65.2 | 76.8 | 3 | 1 | 0 | 1 | 1 | 1 |
| Specimen 2 | 7.0 | 18.5 | 1 | 1 | 0 | 0 | 0 | 0 |

The results shown in Table 2 demonstrate that the reagent using ΔTpN17 deprived of a part of the amino acid sequence of complete TpN17 exhibits a higher value than the reagent using TpN17 deprived of the amino acid sequence of the signal peptide and the lipid-modified cysteine residue of the amino acid sequence of complete TpN17. The results also demonstrate that the former enables detection of antibodies with higher sensitivity.

The results of detection of various *anti-Treponema pallidum* antibodies in the serum specimens indicate that Specimen 1 contains IgM and IgG and Specimen 2 contains IgM.

In comparison with Specimen 1, Specimen 2 exhibits a larger difference in measurement values between the reagent using ΔTpN17 deprived of a part of the amino acid sequence of complete TpN17 and the reagent using TpN17 deprived of the amino acid sequence of the signal peptide and the lipid-modified cysteine residue of the amino acid sequence of complete TpN17. This indicates that the protein of the present invention comprising a sequence deprived of a part of the amino acid sequence of TpN17 responds to the anti-TpN17 IgM antibody that appears at a very early stage in the patient's serum with high sensitivity and enables detection of the antibody of the syphilis patient at an early stage.

### Industrial Applicability

The method of the present invention enables detection of syphilis infection at an early stage of infection.

### Sequence Listing Free Text

### SEQ ID NO: 3: Synthetic

All publications, patents, and patent applications cited herein are incorporated herein by reference in their entirety.

## Claims

1. A protein comprising an amino acid sequence of TpN17, comprising deletion of a part of the amino acid sequence thereof.

2. The protein according to claim 1, wherein the deletion of a part of the amino acid sequence of TpN17 is deletion of 1 to 20 amino acids at the N terminus of the amino acid sequence shown in SEQ ID NO: 1.

3. The protein according to claim 2 comprising the amino acid sequence shown in SEQ ID NO: 3, wherein the deletion of a part of the amino acid sequence of TpN17 is deletion of 11 amino acids at the N terminus.

4. A TpN17 antigen comprising the protein according to any one of claims 1 to 3.

5. A method for measuring an *anti-Treponema pallidum* antibody by an immunoassay method with the use of the protein according to any one of claims 1 to 3 as the antigen.

6. A method for measuring an *anti-Treponema pallidum* antibody by an immunoassay method with the use of the TpN17 antigen according to claim 4 as the antigen.

7. The method according to claim 5, comprising measuring an *anti-Treponema pallidum* IgM antibody with high sensitivity.

8. The method according to claim 6, comprising measuring an *anti-Treponema pallidum* IgM antibody with high sensitivity.

9. The method according to claim 5, which is an agglutination method.

10. The method according to claim 6, which is an agglutination method.

11. The method according to claim 7, which is an agglutination method.

12. The method according to claim 8, which is an agglutination method.

13. A kit for measuring an anti-*Treponema pallidum* antibody by an immunoassay method, the kit comprising the protein according to any one of claims 1 to 3 as an antigen for measurement.

14. A kit for measuring an *anti-Treponema pallidum* antibody by an immunoassay method, the kit comprising the TpN17 antigen according to claim 4 as an antigen for measurement.

15. The kit according to claim 13, wherein the immunoassay method is an agglutination method.

16. The kit according to claim 14, wherein the immunoassay method is an agglutination method.
